# EUROPEAN PATENT SPECIFICATION

(11) **EP 2 402 357 B1**
(45) Date of publication and mention of the grant of the patent: **14.10.2015**
(21) Application number: 10172852.5
(22) Date of filing: 15.08.2010
(51) Int. Cl.: C07H 19/10, A61K 31/7068, A61P 31/18

(54) **Analogs of 2',3'-dideoxy-3'-fluorothymidine 5'-monophosphate (FLTMP) for use in the treatment of infections caused by human HIV-1 strains**
Analoga von 2',3'-Dideoxy-3'-fluorthymidin 5'-monophosphat (FLTMP) zur Verwendung bei der Behandlung von Infektionen, die von humanen HIV-1 Stämmen verursacht werden
Analogues de 2',3'-dideoxy-3'-fluorothymidine 5'-monophosphate (FLTMP) pour l'utilisation dans le traitement des infections provoquées par souches humaines de VIH-1

(30) Priority: 30.06.2010 PL 39167610
(43) Date of publication of application: 04.01.2012
(73) Proprietor: Instytut Biochemii i Biofizyki PAN, 02-106 Warszawa (PL)
(72) Inventor: MIAZGA, Agnieszka, 37-400, Nisko (PL); ZIEMKOWSKI, Przemyslaw, 03-130, Warszawa (PL); KULIKOWSKI, Tadeusz, 02-116, Warszawa (PL); HAMY, Francois, F-68110, Illzach (FR); LOUVEL, Severine, F-68220, Hagenthal le haut (FR); KLIMKAIT, Thomas, D-70539, Loerrach (DE)
(74) Representative: Brodowska, Iwona

(56) References cited:
- PL-B1- 197 669
- MIAZGA A. ET AL: "Synthesis, Biological Properties and Anti-HIV-1 Activity of New Pyrimidine P1,P2-Dinucleotides", NUCLEOSIDES, NUCLEOTIDES AND NUCLEIC ACIDS, vol. 29, no. 4-6, June 2010 (2010-06), pages 438-444, XP002627045, DOI: 10.1080/15257771003738642
- MATTHES E ET AL: "Inhibition of HIV-replication by 3'-fluoro-modified nucleosides with low cytotoxicity", BIOCHEMICAL AND BIOPHYSICAL RESEARCH COMMUNICATIONS, ACADEMIC PRESS INC. ORLANDO, FL, US, vol. 165, no. 1, 30 November 1989 (1989-11-30), pages 488-495, XP024842023, ISSN: 0006-291X, DOI: DOI:10.1016/0006-291X(89)91096-6 [retrieved on 1989-11-30]
- MIAZGA ET AL: "Thiated Analogues of 2,3-Dideoxy-3-fluorothymidine and Their Phosphorylated and Phosphonylated Derivatives: Synthesis, Interaction with HIV Reverse Transcriptase, and In Vitro Anti-HIV Activity", NUCLEOSIDES & NUCLEOTIDES, MARCEL DEKKER, INC, US, vol. 22, no. 5-8, 1 January 2003 (2003-01-01), pages 973-975, XP009163240, ISSN: 0732-8311
- FERRIS R G ET AL: "Antiviral activity of GW678248, a novel benzophenone nonnucleoside reverse transcriptase inhibitor", ANTIMICROBIAL AGENTS AND CHEMOTHERAPY, AMERICAN SOCIETY FOR MICROBIOLOGY, US, vol. 49, no. 10, 1 October 2005 (2005-10-01), pages 4046-4051, XP002376573, ISSN: 0066-4804, DOI: 10.1128/AAC.49.10.4046-4051.2005 & NUCLEOSIDES NUCLEOTIDES & NUCLEIC ACIDS, vol. 29, no. 4-6, 2010, pages 438-444, ISSN: 1525-7770, DOI: 10.1080/15257771003738642

## Description

The present invention relates to analogs of 3'-fluorothymidine 5'-monophosphate (FLTMP) represented by the **Formula 1,** 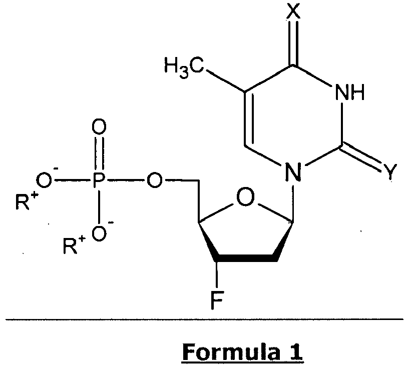 where **X** and **Y** are the same or different and means independently sulphur atom or oxygen atom, at least one designation means sulphur atom, and **R** are the same or different means cation chosen from the group including Li⁺, Na⁺, K⁺ and N⁺(CH₂CH₃)₃, with low toxicity, and their medical application as an active substances inhibiting the replication of human immunodeficiency virus (HIV-1) drug, and multidrug resistant (MDR) strains.

The invention relates also to pharmaceutically acceptable forms of drug against drug and multidrug resistant (MDR) HIV-1 strains comprising these analogs, applied at antiviral therapeutic doses, favorably as water solutions suitable for oral administration.

The present invention relates also to new application of 2- and 4-thio substituted analogs of 2',3'-dideoxy-3'-fluorothymidine 5'-monophosphate, represented by the **Formula 1** namely in the manufacture of a medicament for the treatment of drug and multidrug resistant immunodeficiency virus (HIV-1) strains infections.

HIV-1 virus is of particular importance: it is highly pathogenic member of *Retroviridae* family, widely distributed all over the world (according to WHO 2008 report ca. 40 million infected people). Persistent HIV infection leads to deadly disease - acquired immunodeficiency syndrome (AIDS).

Since the discovery of HIV/AIDS, numerous trials have been made for anti-HIV chemotherapeutic agents. HIV nucleoside reverse transcriptase inhibitors (NRTIs) present the most important class of drugs employed in multidrug highly active antiretroviral therapy (HAART). Unfortunately these drugs when employed in long-term anti-HIV therapy develop multidrug resistance (MDR). At present the emergence of HIV-1 variants resistant to standard drugs is one of the major obstacles to chemotherapy of HIV-1 infection. HIV-1 can develop multidrug resistance in patients receiving various combined chemotherapies. Therefore the development of novel compounds that are active against multidrug resistant HIV-1 variants, are urgently needed. [De Clercq, Nat. Rev. Drug Discovery 2002, 1, 13-25; Youcef and De Clercq, J.Med.Chem. 2010, 53, 521-538]. Ferris et al. [Antimicrob. Agents Chemother. 2005, 49, 4046-4051] described inhibition of drug and multidrug resistant HIV-1 strains by GW678248 - the benzophenone derivative.

2',3'-Dideoxy-3'-fluorothymidine (FLT, alovudine^{®}) belongs to the most potent agents inhibiting HIV replication. Its 5'-triphosphate (FLTTP) is potent inhibitor of HIV-1 reverse transcriptase (HIV RT). Moreover the development of HIV mutants resistant to FLT is much slower than for the other HIV RT inhibitors. However FLT shows some citotoxicity in both cell cultures [Balzarini, Baba, Pauwels, Herdwijn, DeClercq, Biochem.Pharmacol. 1998, 37, 2847-2856; Matthes, Lehman, Scholz, Rosenthal, Langen, Biochem.Biophys.Res.Commun. 1988, 153, 825-831], and also *in vivo* in man [Kong, Zhu, Vidal, Watanabe, Polsky, Armstrong, Ostrander, Lang, Muchmore, Chou, Antimicrob.Agents.Chemother., 1992, 36, 808-818]. These adverse side effects prompted previous authors to synthesize thiated at positions 2 and 4 analogs of FLT. [Matthes et al., Biochem. Biophys. Res. Commun. 1989, 165, 488-495; Poopeiko et al., Nucleosides Nucleotides and Nucleic Acids 1995, 14, 435-437]. It was previously suggested that 5'-triphosphates of antiviral nucleoside analogs do not penetrate the cells [DeClercq et al., Biochem. Pharmacol. 1980, 29, 2883 - 2885], or penetrate to small extent [Le Page et al., Cancer Res. 1975; Cohen S.S. Biochem. Pharmac. 1975, 24, 1929; Plunkett et al., Proc. Nat. Acad. Sci. USA 1974, 71, 73].

Lipophylic analogs of FLT, 2',3'-dideoxy-3'-fluoro-2-thiothymidine (S2FLT) and 2',3'-dideoxy-3'-fluoro-4-thiothymidine (S4FLT), as already reported by us [Miazga et al., Nucleoside Nucleotides 2003 22, 973-975; Miazga et al., Nucleoside Nucleotides 2010, 29, 438-444] potently inhibit HIV-1 *in vitro* with low citotoxicity.

The inventors surprisingly discovered, that 2- and 4-thio derivatives of 3'-fluorothymidine-5'-monophosphate according to the invention, exert potent inhibitory activity against HIV-1 virus strains resistant to so far applied drugs.

The inventors unexpectedly discovered that the compounds exert potent inhibition of replication of HIV-1 wild type, as well as of M184V, K103N, K65R, M4 drug resistant mutants and even of multidrug resistant strain GQ using the replicative phenotyping assay deCIPhR Louvel et al., HIV Med. 2008, 9, 133-141; Opravil et al., J. Acquir. Immune Defic. Syndr. 2010, 54, 51-58]. Finally, the inventors showed that 4-SFLT 5'-monophosphate exhibit high selectivity.

Analogs of 3'-fluorothymidine 5'-monophosphate (FLTMP), represented by the **Formula 1**, 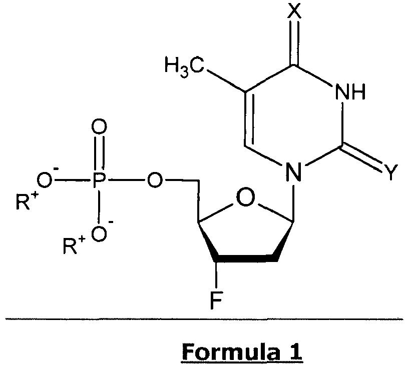 where **X** and **Y** are the same or different and means independently sulphur atom or oxygen atom, at least one designation means sulphur atom, and **R** are the same or different and means a cation chosen from a group including Li⁺, Na⁺, K⁺ and N⁺(CH₂CH₃)₃ group.

Analogs of the invention, preferably 3'-fluoro-2-thiothymidine 5'-monophosphate intended 2',3'-dideoxy-3'-fluoro-β-D-*erythro* -pentofuranoside of 2-thiothymine 5'-monophosphate (2-SFLTMP); by 3'-fluoro-4-thiothymidine 5'-monophosphate intended 2',3'-dideoxy-3'-fluoro-β-D-*erythro*-pentofuranoside of 4-thiothymine 5'-monophosphate (4-SFLTMP).

Particularly favorable FLTMP analogues are compounds of **Formula 1**, where **X** and **Y** are the above-mentioned meaning, and **R** is the lithium atom or sodium atom, and especially dilithium salt.

Favorably, pharmaceuticals by invention is in the form of aqueous solutions.

These compounds, because of their low toxicity, may be used in all steps of AIDS development, also in the final stage, i.e. when the amount of T₄ lymphocytes drops down below 200/µL in peripheral blood.

This invention also includes pharmaceutical acceptable form of drug, which according to the invention, contain known acceptable media and auxiliary substances, containing, as an active agents analogues of general **Formula 1**, in which **X**, **Y** and **R** have the above-mentioned meaning, especially dilithium salt of 2',3'-dideoxy-3'-fluoro-β-D-*erythro*-pentofuranoside of 2-thiothymine 5'-monophosphate 2-SFLTMP and dilithium salt 2',3'-dideoxy-3'-fluoro-β-D-*erythro*-pentofuranoside of 4-thiothymine 5'-monophosphate 4-SFLTMP.

This invention includes also medical use of 2-and 4-thiosubstituted analogues of 3'-fluorothymidine 5'-monophosphate, according to the invention, represented by the **Formula 1,** wherein **X, Y** and **R** have the abovementioned meaning, in the manufacture of a medicament, for the treatment infections caused by the human immunodeficiency virus (HIV-1) wild type strains and those showing drug and multidrug resistance.

The inventors unexpectedly discovered that proposed new FLTMP analogs with thiosubstituent at 2 or 4 position of FLT pyrimidine ring and monophosphate group at 5'-O-of 2,3-dideoxyribose exert very potent inhibitory activity against multidrug resistant HIV-1 strains, as well as high selectivity and good solubility in water. Particularly favourable are FLTMP analogs represented by the general **Formula 1**, wherein **R** represents lithium or sodium cation.

Abovementioned analogs of the invention exhibit very potent inhibition of replication of M184V, K103N, K65R, M4 HIV-1 virus drug resistant mutants as well as of multidrug resistant GQ strain in the deCIPhR™ cellular system, showing moreover high selectivity of 4-SFLT 5'-monophosphate.

Abovementioned pharmaceuticals, according to the invention show potent inhibition of replication of HIV-1 virus drug resistant mutants M184V, K103N, K65R, M4 and MDR strain GQ in the cellular system deCIPhR™ showing moreover high selectivity of 4-SFLTMP. **Table 1** shows antiviral activity (EC₅₀ and EC₉₀) of thiated nucleoside 5'-monophosphates in cellular system deCIPhR™.

### General procedure for the synthesis of nucleoside 5'-monophosphates.

Analogues of the invention shall be obtained by adding to an ice-cooled solution of dried 1,2,4-triazole in dry 1,4-dioxan, POCl₃ and a solution of triethylamine in 1,4-dioxan was added. After stirring for 1 h at room temperature the reaction mixture was directly filtrated into a flask containing nucleoside. Phosphorylation of nucleoside was completed in 15 min and then H₂O was added. The mixture was left to stand overnight at room temperature. After this time the solvent was removed and the product was purified by column chromatography on a DEAE-Sephadex A-25 eluted with a linear gradient of triethylammonium hydrogen carbonate (TEAB) buffer. The fractions containing pure monophosphate were pooled and lyophilized to remove excess bicarbonate buffer to yield nucleoside 5'-phosphate. The product was dissolved in a small amount of water and 5 % solution of Nal in acetone was added dropwise. The formed precipitate was filtered off, washed several times with acetone and dried.

The examples are presented below which illustrate the invention.

### Example I.

### 1-(2-deoxy-3-fluoro-β-D-ribofuranosyl)-2-thiothymine 5'-monophosphate disodium salt.

To an ice-cooled solution of dried 1,2,4-triazole (314 mg, 4.4 mmol) in 8 mL of dry 1,4-dioxan, POCl₃ (150 µL, 1.6 mmol) and a solution of triethylamine (0.62 mL, 4.44 mmol) in 2 mL of 1,4-dioxan was added. After stirring for 1 h at room temperature the reaction mixture was directly filtrated into a flask containing nucleoside (1 mmol). Phosphorylation of nucleoside was completed in 15 min and then 0.5 mL of H₂O was added. The mixture was left to stand overnight at room temperature. After this time the solvent was removed and the product was purified by column chromatography on a DEAE-Sephadex A-25 eluted with a linear gradient of triethylammonium hydrogen carbonate (TEAB) buffer (1M, pH 7.5) (0 to 0.4 M). The fractions containing pure monophosphate were pooled and lyophilized to remove excess bicarbonate buffer to yield nucleoside 5'-phosphate. The product was dissolved in a small amount of water and 5 % solution of Nal in acetone was added dropwise. The formed precipitate was filtered off, washed several times with acetone and dried.
Yield 276 mg (75 %); TLC (cellulose) R_{f} (i-PrOH - conc. NH₃-H₂O 70:10:20) 0.15; ¹H NMR δ [ppm] (D₂O) 8.03 (1H, psd, H6), 7.13-7.10 (1H, dd, H1'), 5.51-5.39 (1H, dd, H3', *J_{3',F}* = 52.5 Hz), 4.61-4.55 (1H, m, 4', *J_{4',F}* = 27.5 Hz), 4.13-4.05 (2H, m, H5', H5"), 2.87-2.79 (1H, m, H2"), 2.36-2.22 (1H, m, H2'), 1.97 (3H, s, CH3); ³¹P NMR δ [ppm] (D₂O) 1.66; MS *m*/*z* 339 (M-Na)⁻

### Example II.

### 1-(2-deoxy-3-fiuoro-β-D-ribofuranosyl)-4-thiothymine 5'-monophosphate disodium salt.

Using the same method as in example 1, we obtain:
Yield 250 mg (65 %); TLC (cellulose) R_{f} (i-PrOH - conc. NH₃ - H₂O, 70:10:20) 0.15, ¹H NMR δ [ppm] (D₂O) 7.92 (1H, s, H6), 6.37 (1H, dd, H1') 5.51-5.36 (1H, dd, H3', *J*_{3',F} = 53 Hz), 4.56-4.49 (1H, m, H4', *J*_{4',F} = 27 Hz), 4.07-3.96 (2H, d, H5', H5"), 2.71-2.6 (1H, m, H2"), 2.49-2.3 (1H, m, H2'), 2.1 (3H, s, CH₃); ³¹P NMR δ [ppm] (D₂O) 3.95; MS *m*/*z* 339 (M-Na)⁻.

### Example III.

### 1-(2-deoxy-3-fluoro-β-D-ribofuranosyl)-thyrmine 5'-monophosphate disodium salt.

Using the same method as in example 1, we obtain:
Yield 173 mg (47 %); TLC (cellulose) R_{f} (i-PrOH - conc. NH₃ - H₂O, 70:10:20) 0.14, ¹H NMR δ [ppm] (D₂O) 7.92 (1H, s, H6), 6.53 (1H, dd, H1') 5.63-5.48 (1H, dd, H3', *J*_{3',F} = 52.7Hz), 4.67-4.6 (1H, m, H4', *J*_{4',F} = 27.3 Hz), 4.23-4.14 (2H, m, H5', H5"), 2.79-2.68 (1H, m, H2"), 2.58-2.41 (1H, m, H2'), 2.03 (3H, s, CH₃); ³¹P NMR δ [ppm] (D₂O) 0.75.

### Example IV.

Antiviral activity (EC₅₀ and EC₉₀) and citotoxicity (CD₅₀) of obtained (Examples I, II, III) thiated nucleoside 5'-monophosphates were determined in the deCIPhR™ cellular system with use of following methods.

The compounds show potent inhibition of replication of HIV-1 virus wild type as well as of resistant mutants M184V, K103N, K65R, M4 and multidrug resistant strain GQ in the deCIPhR™ cellular system showing moreover high selectivity of 4-SFLTMP. **Table 1** shows antiviral activity (EC₅₀ and EC₉₀) of 2- and 4-thiated nucleoside 5'-monophosphates in deCIPhR™ cellular system.

### Determination of antiviral activity

Fully infectious virus HIV-1 was produced from cloned proviral DNA reference coding either for wild-type HIV-1 or from drug-resistant virus following transfection of proviral DNA into recipient mammalian cells. Subsequent to viral protein expression and particle formation, culture supernatant was harvested and used to infect an indicator cell line. This latter cell line expresses all necessary receptors and co-receptors for HIV infection as well as the bacterial gene LacZ coding for β-galactosidase under the control of the HIV-1 long terminal repeat. In this system, amount of β-galactosidase is directly proportional to the extent of viral replication. Activity of β-galactosidase is assayed in presence of a colorless substrate which upon enzymatic conversion yields a yellow product absorbing at 405 nm. Next, variation in colorimetric readout in presence of test compounds was translated in percent viral inhibition following normalization according to absorbance values of positive (presence of anti-HIV drug) and negative (untreated) control wells included in each 96 well-plate. For determining effective concentration 50 % (EC₅₀), test compounds were assayed across a range of concentrations. Percent inhibition data in function of drug concentration were processed by a statistical curve fitting software yielding a modeled curve from which EC₅₀ was extrapolated.

### Determination of cytotoxicity: Alamar Blue™ Cell Viability Assay

The Alamar Blue™ Cell Viability Assay (Invitrogen) is a fluorimetric metod for determining the number of viable cells. The Alamar Blue Reagent contains the cell permeable non-fluorescent blue dye resazurin, which is converted to the pink and fluorescent dye resorufin in response to the action of reductases located in the cytosol, mitochondria and endoplasmic reticulum. Therefore, only living cells will convert resazurin into resorufin. Assays are performed by adding 20 µL of the Alamar Blue Reagent directly to culture wells followed by a 6-hour incubation at 37°C. The fluorescent signal is monitored using 545 nm excitation wavelength and 590 nm emission wavelength. The quantity of resofurin product as measured by fluorescence is directly proportional to the number of living cells in culture. Determined CD₅₀ value was 20.000±0.177 µM.

**Table 1. Antiviral activity (EC₅₀ and EC₉₀) of thiated nucleoside 5'-monophosphates in deCIPhR™ ¹ cellular system**

| Strain HIV-1 | EC₅₀ µM (EC₉₀ µM) | | |
|---|---|---|---|
| | FLT² | 4-SFLTMP | 2-SFLTMP |
| wild type pNL4-3 | 0.021 (0.210) | 0.024 (0.166) | 0.224 (2.584) |
| mutant M184V | 0.027 (0.119) | 0.043 (0.425) | 0.144 (3.26) |
| mutant K103N | 0.018 (0.071) | 0.011 (0.050) | 0.114 (0.978) |
| mutant K65R | 0.032 (0.096) | 0.096 (0.338) | 0.504 (4.335) |
| mutant M4 | 0.016 (0.118) | 0.031 (0.381) | 0.392 (3.683) |
| mutant GQ (MDR) | 0.131 (1.295) | 0.048 (1.800) | 2.850 (4.532) |

| | | | |
|---|---|---|---|
| ¹ F. Hamy et all., InPheno Study Report #0926-7 ² Reference compound | | | |

## Claims

1. Analogs of 3'-fluorothymidine 5'-monophosphate (FLTMP) represented by **Formula 1,** 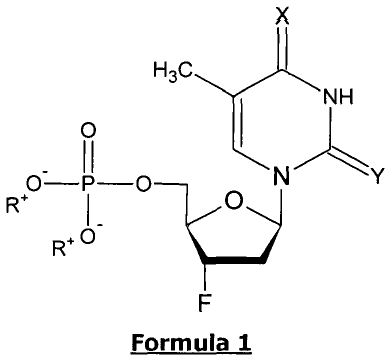 where **X** and **Y** are the same or different and means independently sulphur atom or oxygen atom, at least one designation means sulphur atom, and R are the same or different and means cation chosen from group including Li⁺, Na⁺, K⁺ and N⁺(CH₂CH₃)₃-group, for use in the treatment of infections caused by human immunodeficiency virus (HIV-1) strains showing drug resistance and multidrug resistance.

2. Compounds according to claim 1, wherein the HIV-1 strains are resistant mutants M184V, K103N, K65R, M4 and multidrug resistant GQ.

## Patentansprüche

1. Analoge 3'-fluorthymidin-5'-monophosphat (FLTMP) dargestellten Formel 1, 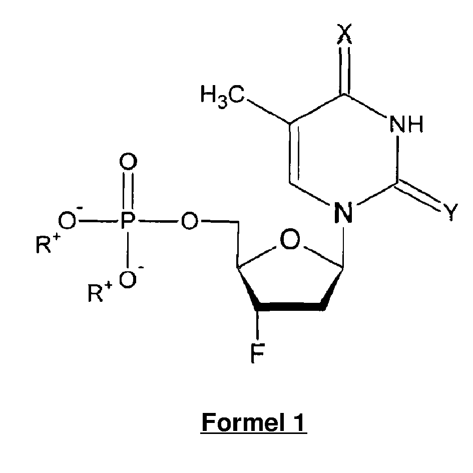 wobei X und Y gleich oder verschieden ist und unabhängig ein Schwefelatom oder Sauerstoffatom, zumindest auf Bezeichnung bedeutet Schwefelatom darstellt und R dasselbe oder verschieden ist und Kation ausgewählt aus der Gruppe einschließlich Li⁺, Na⁺, K⁺ und N⁺(CH₂CH₃)₃ Gruppe, zur Verwendung in der Behandlung von Infektionen durch humanen Immunschwächevirus verursacht (HIV-1) Stämme, Resistenzen und Multidrug-Resistenz.

2. Verbindungen nach Anspruch 1, wobei die HIV-1-Stämme sind resistent Mutanten M184V, K103N, K65R, M4 und Multidrug-resistenten GQ.

## Revendications

1. Des analogues de 3'-fluorothymidine-5'-monophosphate (FLTMP) représentaient la formule 1, 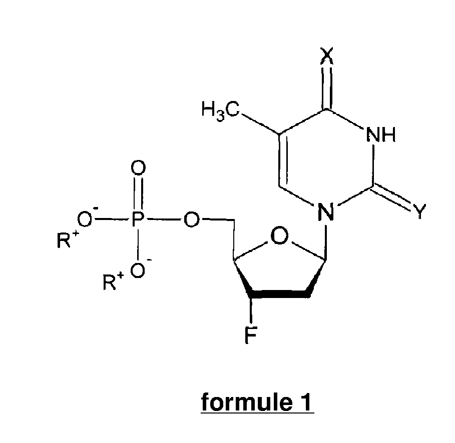 où X et Y sont identiques ou différents et désigne indépendamment un atome de soufre ou un atome d'oxygène, au moins sur désignation désigne un atome de soufre, et R sont identiques ou différent et signifie cation choisi dans le groupe comprenant Li⁺, Na⁺, K⁺ und N⁺(CH₂CH₃)₃ groupe, pour utilisation dans le traitement d'infections causées par le virus de l'immunodéficience humaine (VIH-1) souches présentant une résistance aux médicaments et de la résistance à des médicaments multiples.

2. Composés selon la revendication 1, dans lequel les souches de VIH-1 sont des mutants résistants M184V, K103N, K65R, M4 et GQ multidrogue résistant.
